# NEW EUROPEAN PATENT SPECIFICATION

(11) **EP 0 244 234 B2**
(45) Date of publication and mention of the opposition decision: **07.11.2001**
(45) Mention of the grant of the patent: 21.07.1993
(21) Application number: 87303834.3
(22) Date of filing: 29.04.1987
(51) Int. Cl.: C12N 15/52, C12N 1/14, C12N 15/80, C12N 9/42, C12N 9/64

(54) **Transformation of trichoderma**
Transformation von Trichoderma
Transformation de trichoderma

(30) Priority: 30.04.1986 GB 8610600
(43) Date of publication of application: 04.11.1987
(73) Proprietor: Altia Group Ltd, 00180 Helsinki (FI)
(72) Inventor: Knowles, Jonathan, 1211 Grand Saconnex (CH); Harkki, Anu Marjukka, 02320 Espoo 32 (FI); Nevalainen, Helena, 02230 Espoo (FI); Penttilä, Merja, 00390 Helsinki (FI); Hansen, Mogens Trier, 3540 Lynge (DK)
(74) Representative: Bassett, Richard Simon

(56) References cited:
- EP-A- 0 215 594
- GENE, vol. 26, 1983, pages 205-221, Elsevier Science Publishers; J. TILBURN et al.: "Transformation by integration in Aspergillus nidulans"
- GENE, vol. 36, no. 3, 1985, pages 321-331, Elsevier Science Publishers, Amsterdam, NL; D.J. BALLANCE et al.: "Development of a high-frequency transforming vector for Aspergillus nidulans"
- GENE, vol. 37, 1985, pages 207-214, Elsevier Science Publishers; F.P. BUXTON et al.: "Transformation of Aspergillus niger using the argB gene of Aspergillus nidulans"
- BIOTECHNOLOGY, vol. 4, no. 12, December 1986, pages 1057-1062, Nature Publishing, New York, US; J.V. BRUNT: "Fungi: the perfect hosts?"

## Description

The present invention relates to a vector system for use in the transformation of Trichoderma, a process for high level expression and secretion of proteins in Trichoderma, DNA recombinant vectors and transformed Trichoderma strains.

### BACKGROUND OF THE INVENTION

Filamentous fungi are lower eukaryotes widely used in biotechnology to make various fermentation products. Fungi secrete many industrially important enzymes such as glucoamylase, proteases, lactase, pectinases and glucose oxidase.

Filamentous fungi have a number of biotechnical advantages. They generally produce high amounts of proteins, cultivation in large scale is not complicated and separation of the mycelium from the culture liquid after the fermentation is easy.

The mesophilic imperfect fungus Trichoderma reesei (formerly T. viride) (ref. 1) produces enzymes needed in conversion of cellulosic biomass and is probably the most widely investigated of all cellulase-producing organisms.

For hydrolysis of cellulose to glucose, three types of enzyme activity are needed: randomly cleaving endoglucanases (1,4-β-D-glucan glucanohydrolase, EC 3.2.1.4) which usually attack substituted soluble substrates and show no activity to crystalline cellulose; cellobiohydrolase (1,4-β-D-glucan cellobiohydrolase, EC 3.2.1.91) capable of degrading crystalline cellulose but having no activity towards derivatized cellulose and β-glucosidase (β-D-glucoside glucohydrolase, EC 3.2.1.21) attacking cellobiose and cello-oligosaccharides to yield glucose. Synergistic action between some of these enzymes has been demonstrated (refs. 2-4).

Fungal cellulases have been purified and characterized and all three main types of enzymes have been shown to occur in multiple forms (ref. 5). Two immunologically distinctive cellobiohydrolases, CBH I and CBH II have been detected from the culture medium of T. reesei (refs. 4, 6). Five to eight electrophoretically distinct endoglucanases have been reported, many of them showing varying substrate specificities (refs. 7, 8, 9, 10). Characterization of two extracellular β-glucosidases has been reported (ref. 11).

Intensive strain development using the direct approach of mutation and screening has successfully produced several high-yielding T. reesei mutant strains (refs. 12-22). The amount of extracellular protein produced by the best T. reesei mutants is as much as 20-30 g/litre of culture fluid which is more than 50 % of the total cell protein. A major part of the secreted protein comprises cellulases among which the CBH I component is most abundant, representing up to 60 % of the secreted cellulase proteins.

The gene for CBH I has been cloned by Shoemaker et al, (ref. 23) and Teeri et al. (ref. 24) and the whole nucleotide sequence of the gene has been published (ref. 23). From T. reesei, also the gene for the major endoglucanase EG I has been cloned and characterized (ref. 25, 26, 27). Other isolated cellulase genes are cbh2 (ref. 28, 29) and eg13 (ref. 30).

The molecular biology of industrially important filamentous fungi is in general not well known. This is partly due to the lack of the sexual reproduction cycle and/or genetical-transformation system. Recently, transformation systems have been developed for Neurospora crassa (ref. 31), A. nidulans (refs. 32, 33, 34) and A. niger (ref. 35) or Trichoderma (ref. 61) generally having their basis in complementation of the mutant host by respective functional gene carried by the vector molecule. However, of these fungi only A. niger is of industrial interest at the moment.

In the classification of fungi, the genus Aspergillus is included in the class Ascomycetes, sub-class Euascomycetes. Euascomycetes are divided into three groups, Plectomycetes, Pyrenomycetes and Discomycetes on the basis of the fruiting bodies. The most important genera are Aspergillus and Penicillium - (ref. 49). Trichoderma, instead, is classified as a member of Fungi imperfecti. Fungi imperfecti is a catch-all cathegory of fungi which have no sexual reproduction or obvious affinities with sexually reproducing genera, such as the highly characteristic Aspergillus. Although Trichoderma has been reported to possess a poorly defined sexual stage being an imperfect state of the perfect ascomycete species Hypocrea (ref. 50), the genera Aspergillus and Trichoderma are clearly to be considered taxonomically very different. It has also been shown that the argB gene from Aspergillus nidulans and the pyr4 gene from Neurospora crassa do not hybridize, under non-stringent conditions, to the respective Trichoderma genes thus supporting the idea that Trichoderma is evolutionary quite distinct from other Ascomycetes (Vanhanen, S., in preparation).

Due to its exceptional ability to secrete proteins into the growth-medium Trichoderma reesei is a good candidate as a possible host for the production of proteins. However, genetic studies of T. reesei have so far been directed almost exclusively to improve the cellulase-producing properties of the fungus and the only technique used for the development of hypercellulotic Trichoderma strains has been traditional mutagenesis and screening. No transformation systems giving stable transformants in for Trichoderma have so far been developed.

It is the main object of the present invention to provide Trichoderma stably transformed with an effective host-vector system to obtain a high level expression and secretion of hetecrologous proteins in Trichoderma or to enhance the production of homologouse proteins.

In the present specification with claims, the expression "proteins heterologous to Trichoderma" menus proteins not naturally produced by Trichoderma whereas "proteins homologous to Trichoderma" means proteins produced by Trichoderma itself.

In the fungal transformation systems first developed, namely for Aspergillus and Neurospora, transformation is carried out using protoplasts. The transforming DNA is usually integrated into the host genome. To provide a selection system for identifying stable transformants the vector system must carry a functional gene (a selection marker) which either complements a corresponding mutation of the host genome or supplies an activity, usually an enzyme, required for the growth of the prototrophic strain on a particular growth medium.

### SUMMARY OF THE INVENTION

The present invention describes Trichoderma strains stably transformed with a recombinant DNA cloning vector system. When the DNA cloning vector system comprises a DNA-sequence encoding a desired protein product transformation of Trichoderma with the present vector system will provide a high level expression and secretion of the desired protein when the transformed microorganism is grown in a suitable culture medium.

According to its first aspect the present invention provides a Trichoderma strain stably transformed with a vector system comprising:
a) a gene encoding a desired protein product,
b) functions facilitating gene expression including DNA sequences comprising promoters/enhancers operably linked to said gene to control expression of the desired product, and
c) a selection marker capable of selecting stable Trichoderma transformants, the selection marker not being derived from the Neurospora crassa pyr-4 gene.

According to its second aspect the present invention provides a method for transformation of

Trichoderma, wherein a suitable Trichoderma strain is transformed with the present vector system.

According to its third aspect the present invention provides a method for the production of a protein in Trichoderma which comprises transforming Trichoderma with the present vector system, culturing the transformed strain in a suitable medium and recovering the expressed and secreted product from the medium.

The present invention also provides a method for the production of a protein in Trichoderma by which method a Trichoderma strain transformed with the vector system is cultivated in a suitable culture medium and the expressed and secreted protein is recovered from the culture medium.

As used herein the expression "vector system" includes a single vector or plasmid or two or more vectors or plasmids which together contain the DNA-information to be integrated into the host genome. The vectors or plasmids may be linear or closed circular molecules. Although self-replicating plasmids in Trichoderma are not known at present the invention also covers such self-replicating plasmids if they should be found at a later time.

The vector system comprises DNA-sequences encoding functions facilitating gene expression including promoters, enhancers and transcription initiation sites as well as terminators, a marker for selection of transformants and a DNA-sequence encoding the desired protein.

To ensure secretion of the expressed product the gene for the desired product is preferably provided with a preregion ensuring effective direction of the expressed product into the secretory pathway of the cell. This preregion which might be a naturally occuring signal or leader peptide or parts thereof is generally cleaved off from the desired product during secretion whereupon the mature product can be isolated from the culture medium.

The gene for the desired product may be obtained from genomic clones or cDNA clones. DNA-sequences may also be synthesized.

The signal/leader sequence may be derived from the signal/leader sequence of the gene encoding the desired protein or may be derived from genes for other secreted proteins either from Trichoderma or from any other source of organism. Examples of signal/leader sequences derived from genes encoding a protein secreted by Trichoderma are the cbh1 signal sequence or the egl1 signal sequence or parts thereof. Signal/leaders sequences derived from genes encoding secreted proteins heterologous to Trichoderma may be derived from genes for Aspergillus amylases or glucoamylase. Also synthetic signal/leader sequences may be used.

The promoter may be any DNA-sequence that shows transcriptional activity in Trichoderma and may be derived from genes either homologous or heterologous to Trichoderma. Examples of promoters derived from genes encoding proteins homologous to Trichoderma are the cbh1 promoter or the egl1 promoter or parts thereof. An example of a promoter derived from a gene for a protein heterologous to Trichoderma Is the Aspergillus glucoamylase promoter. Transcription terminators may be derived from the same sources as the promoters. Also synthetic promoter or terminator sequences may be used.

It is understood and obvious to the expert in the art that all specifically mentioned DNA-sequences may be-modified by amendment or deletion of a couple of bases non-essential to the function of the product encoded for. For example DNA sequences substantially similar to cbh1 or egl1 signal or promoter sequences may be used as long as they exhibit the intended function in Trichoderma. Also the genes for the desired proteins may be altered as long as this has no deleterious, effect on the activity of the protein.

Different selection markers may be used, e.g. argB (A. nidulans or T. reesei), amdS (A. nidulans) trp1 from N.crassa or trpC from A.Nidulans and pyr4 or T. reesei).

The host strain may either he a prototrophic or an auxotrophic Trichoderma strain depending on the selection marker used in the transformation procedure. The amdS gene from A. nidulans may as demonstrated in the experimental part of the specification be used for the transformation of prototrophic T. reesei strains. T. reesei grows poorly on acetamide as the sole nitrogen source and the growth can be further inhibited by adding CsCI to the medium. The growth on acetamide as the sole nitrogen source in the presence of CsCI can accordingly be used as a selection medium to identify AmdS⁺ transformants.

If a selection marker is used which complements a corresponding mutation of the host genome auxotrophic Trichoderma mutants must be constructed. Auxotrophic Trichoderma mutants may be produced by known methods for producing mutant strains.

Auxotrophic Trichoderma mutants requiring uracil, tryptophan or arginine for growth are isolated by a filtration enrichment technique as described by Nevalainen (ref. 36). From arginine requiring auxotrophs, mutant: deficient in the argB gene were identified by using a series of minimal plates supplied by different intermediates in the arginine biosynthesis. From uracil-requiring mutants, strains deficient in the pyr4 gene can be sought for by measuring the orotidine-5'-phosphatedecarboxylase (OMP decase) activity in mycelium preparations (ref. 37).

Mutants having a trpl gene defect can be characterized by the lack of the PRA isomerase - InGP synthetase activity in their mycelia (ref. 39). The trpl -character of the mutants showing no enzyme activity may be confirmed by e.g. transformation and complementation with the N. crassa trpl plasmid (ref. 40) or the trpC -plasmid of A. nidulans (ref. 34).

The transformation technique used is the method adapted from the methods for transformation of A. nidulans (refs. 32, 33). Protoplasts are prepared by known methods by growing mycelium on agar plates and suspending mycelium in a buffered solution of Novozym^{R} 234. Instead of the conventional sucrose solution of Novozym^{R} 234 a sorbitol solution may advantageously be used. Transforming DNA is then added to the protoplast solution as described in further detail in the experimental part of the specification. The transformation is usually carried out as a cotransformation by which a nonselectable plasmid is cotransformed into T. reesei with high frequency using a selectable marker inserted in another plasmid (e.g. amdS in p3SR2 or argB in pSal43). A great proportion of the transformants contain the nonselectable plasmid integrated into the genome if equimolar amounts of DNA are used for transformation. When an argB⁻ T. reesei strain is used in transformation with equimolar amounts of plasmids p3SR2 and pSal43, transformants can be obtained on double selection medium (minimal medium, acetamide, CsCl). Alternatively a single plasmid may be used for transformation in which also the selectable marker is inserted.

Transformants are then cultured in a suitable medium. To be able to produce the desired product in a simple defined medium a glucose inducible promoter may be used. When Trichoderma is grown on a medium containing 1 % glucose, secretion of all extracellular proteins, including proteases is strongly suppressed. When the gene coding for the desired enzyme is connected to a promoter strongly expressed in glucose containing medium, the desired enzyme is secreted to the growth medium. Since other proteins are not produced under these conditions, the desired enzyme is the major protein secreted into the medium.

When the fungal mycelium is removed the desired proteins is present in the resulting solution in a very pure form. If required, it can be very easily further purified, since it is almost the only protein present.

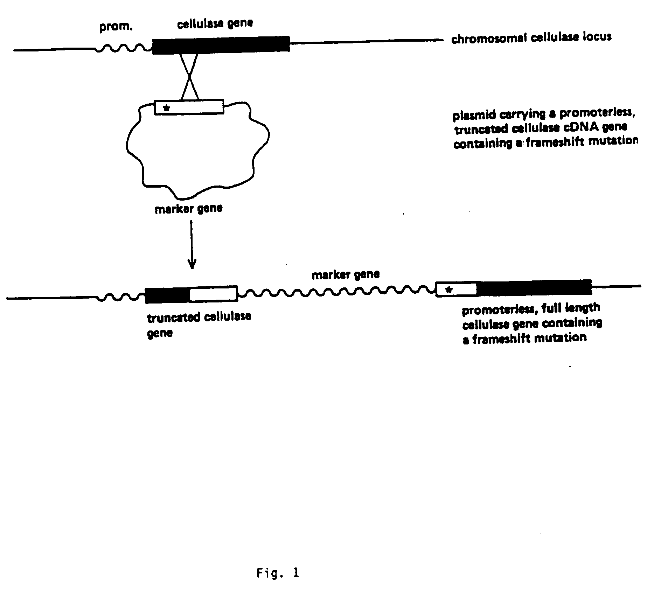

The present invention may also be used to modify or inactivate a gene producing a certain enzyme activity of host organism. As an example cellulase-negative T. reesei strains can he constructed. This mutagenesis is based on transformation of Trichoderma reesei with a plasmid carrying a defected cellulase gene. Homologous recombination of the plasmid at the chromosomal cellulase locus causes insertional inactivation of the endogenous T. reesei cellulase gene. The plasmid used for transformation contains only part of the cellulase coding region and produces inactive protein. No 5' flanking sequences are included. A, frameshift mutation can also be introduced to the truncated coding region. A selection marker (argB) or a marker for screening (lacZ) can he coupled to the plasmid used for transformation. After recombination the marker will be placed between the two resulting defective cellulase genes. Principle of the method is shown in fig. 1.

The present invention is illustrated by means of the production of chymosin and Trichoderma reesei endoglucanase I (EGI). Promoter, signal and leader and terminator sequences were derived from either the glucoamylase gene from Aspergillus niger or the Trichoderma reesei cbh1 gene. As selection marker the amdS gene from A. nidulans was used, as was the argB gene from A. nidulans .

### Materials

| Plasmids: | |
|---|---|
| p285 | ATCC No. 20681 |
| pCAMG91 | ref. 59 |
| pIC19R | refs.60 |
| pSal43 | refs. 51 + 54 |
| p3SR2 | refs. 33 + 35 |
| pDJB2 | ref. 38 |
| pMC1817 | ref. 46 |
| pTT01, pTT11 | ref. 28 |
| pUC9, pUC13 | ref. 61 |
| pUC18, pUC19 | ref. 58 |
| pTZ19R | (Pharmacia) |
| pAN5-41B | ref. 62 |

E. coli strains JM101, JM103, JM109 and and DHI (ref. 51) arc used as hosts in E. coli cloning. Trichoderma reesei strains QM9414 (ATCC 26921) and RUT-C-30 (ATCC 56765) are used used in fungal transformation and expression studies.

| Trichoderma minimal medium: | |
|---|---|
| Glucose | 20 g |
| (NH₄)₂SO₄ | 5 g |
| KH₂PO₄ | 15 g |
| MgSO₄ | 0.6 g |
| CaCI | 0.6 g |
| FeSO₄, 7H₂O | 5 mg |
| MnSO₄, H₂O | 1.56 mg |
| ZnSO₄, 7H₂O | 1.4 mg |
| CoCl₂ | 2 mg / I H₂O |

### Brief description of drawings

The figures of the constructions are not in scale.

Fig. 1 shows the principle of the inactivation of a chromosomal cellulase gene.

Fig. 2 shows the construction of plasmids pMS4 used for insertion mutagenesis of the T. reesei chromosomal Cbh1 locus. Position of a frameshift mutation generated by inactivation of EcoRI site is marked with *.

Fig. 3 shows the construction of plasmid p285' proC.

Fig. 4 shows the construction of plasmid PMT648 and 15 pMT813.

Fig. 5 shows the construction of plasmid pMT837.

Fig. 6 shows the restriction map of plasmid pR27.

Fig. 7 shows the restriction map of plasmid pAMH100.

Fig. 8 shows the construction of plasmid pAMH105.

Fig. 9 shows the construction of plasmids pAMH1103, pAMH1106 and pAMH1101 by loop-mutagenesis using synthesized oligonucleotides OAMH1, OAMH2 and OAMH3.

Fig. 10 shows the linkers NOR 202, NOR 203, OAMH1, OAMH2 and OAMH3.

Fig. 11 shows the restriction maps of plasmids pAMH102 and pAMH104.

Fig. 12 shows the restriction map of plasmid pAMH1103 and the construction of pAMH103.

Fig. 13 shows the restriction map of plasmid pAMH1106 and the construction of pAMH106.

Fig. 14 shows the restriction map of plasmid pAMH1101 and the construction of pAMH101.

Fig. 15 shows the construction of plasmid pAMH110.

Fig. 16 shows the construction of plasmid pAMH111 used for expressionof EGI under Cbh1 promoter function.

Fig. 17 shows the expression of chymosin ink T. reesei, Western blot. Lanes 1; purified prochymosin control, includes traces of pseudochymosin and chymosin. Lane 2; culture supernatant from growth of strain including pAMH102. Lane 3; control supernatant from strain without plasmids. Lane 4; mycelia from strain including pAMH102.

### EXPERIMENTAL PART

### Example 1

### Induction, enrichment and isolation of auxotrophic T. reesei mutant strains.

Auxotrophic mutants requiring uracil, tryptophan or arginine for growth were isolated using filtration enrichment as described by Nevalainen (ref. 36). The mutagenic agent used was UV-light. From arginine requiring auxotrophs, mutants deficient in the argB gene were identified by using a series of minimal plates supplied by different intermediates in the arginine biosynthesis (ref. 36). Mutants requiring citrulline for growth were considered as possible argB⁻ mutants. The argB⁻ character of isolated mutants was confirmed by transforming them into prototrophy using the plasmid pSal43 containing the A. nidulans argB gene (example 4).

From uracil-requiring mutants, strains deficient in the pyr4 gene were sought for. The pyr4⁻ character of these mutants was confirmed by transformation with a plasmid containing the N. crassa pyr4 gene (ref. 38) (example 4).

### Example 2

### Preparation of protoplasts

Mycelium was grown on cellophane disks on Potato Dextrose Agar plates (Difco). Mycelium from 5 cellophane cultures was suspended in 15 ml of a 5 mg/ml solution of Novozym^{R} 234 in 1.2 M sorbitol buffered at pH 5.6 with 0.1 M potassium-phosphate. The mixture was incubated 1.5 - 2 h at 30°C. Protoplasts were separated from mycelial debris by filtration through sintered glass (porosity 1) pelleted for 5 min at 4000 g and washed twice with 1.2 M sorbitol - 10 mM Tris, HCI pH 7.5.

Better purification of protoplasts can be achieved by using a method described by Tilburn et al. (ref. 33). 5 mg/ml solution of Novozym^{R} 234 in 1.2 M MgSO₄ buffered at pH 5.8 was used for proto-plasting. After incubation and filtration the protoplast suspension was centrifugated at 4000 g for 15 min with an overlay of an equal volume of 0.6 M sorbitol - 100 mM Tris, HCI, pH 7.0. Protoplasts formed a sharp band halfway the tube. The protoplasts were suspended in 1 vol of 1.2 M sorbitol - 10 mM Tris, HCI, pH 7.5, spun down and washed twice in 1.2 M sorbitol - 10 mM Tris, HCl, pH 7.5. The protoplasts were suspended in 1.2 M sorbitol - 10 mM CaCl₂ - 10 mM Tris, HCI, pH 7.5 at a concentration of 5 x 10⁶ - 5 x 10⁷ protoplasts/ml.

The viability of the protoplasts was estimated on Trichoderma animal medium with 1 M sorbitol as osmotic stabilizer. The protoplasts were plated in 3 % agar overlay. The regeneration frequency was 40 - 80 % for the strain QM 9414 (ATCC 26921).

### Example 3

### Transformation of T. reesei using A. nidulans acetamidase (amdS) gene.

Plasmid p3SR2 was used in transformation. It contained A. nidulans DNA carrying the whole acetamidase structural gene amdS and its regulatory region amdl (ref. 41 and 33).

20 µl (4 - 10 µg) of transforming DNA was added to 200 µl of protoplast solution: 50 µl of 25 % PEG 6000 - 50 mM CaCl₂ - 10 mM Tris, HCI, pH 7.5 was added and the mixture was incubated for 20 min on ice. 2 ml of the PEG-solution was added and the mixture was further incubated 5 min at room temperature. 4 ml of 1.2 M sorbitol - 10 mM CaCl₂ - 10 mM Tris, HCI, pH 7.5 was added and the protoplasts were plated in 3 % agar overlay. The selective medium was Trichoderma minimal medium with 10 mM acetamide as the sole nitrogen source instead of (NH₄)₂SO₄, and supplemented with 1 M sorbitol as osmotic stabilizer and 12.5 mM CsCI to repress the background growth.

Transformation frequencies from 40 to 600 transformants per *µ*g DNA were obtained for the strain QM 9414 (ATCC 26921). The highest frequencies were obtained when the DNA was purified with two cycles of CsCl/ethidiumbromide centrifugation.

Sporulation was rarely observed on the selective medium but the transformants sporulate normally when transferred to Potato Dextrose Agar. Their ability to grow on acetamide varied. The diameter of the colonies on the selective medium ranged 1 mm to 10 - 20 mm.

The mitotic stability of the transformants was investigated. Ten large transformants of varying size were subcultured on acetamide-CsCI plates, sporulated on potato dextrose (PD) and replated on PD. To exclude heterogeneity caused by heterokaryosis, individual colonies arising from one spore were tested for AmdS⁺ phenotype. One positive clone from each original transformant was subjected to successive platings (5 growth cycles) on non-selective medium and phenotype was tested after each generation on the selective medium. Of 10 large transformants tested, after one cycle on non-selective medium six of the ten transformants gave 100 % AmdS⁺ conidia, three 47-87 % and one transformant gave no AmdS⁺ conidia. This result remained the some through five non-selective growth cycles, suggesting that the initially unstable AmdS⁺ phenotype can be stabilized later on.

From ten original small colonies, three gave spores width variable frequencies of AmdS⁺ phenotype (94 %, 44 % and 5 % of the spores). The other seven clones gave only spores unable to grow on acetamide. Interestingly, all AmdS⁺ spores obtained showed vigorous growth on acetamide plates, characteristic of large colony transformants.

The presence of the plasmid DNA in the transformants was analyzed by Southern blots of total DNA, isolated from transformants (according to Raeder and Broda, ref. 43), cut with Xho I or Sal I and Eco RI. The vector pUC 18 and the Sal I - Eco RI fragment containing the amdS gene of plasmid p3SR2 were used as probes. The transformation was shown to have occurred by recombination at a number of different sites in the Trichoderma genome DNA, one to several copies per genome.

### Example 4

### Transformation of T. reesei with A. nidulans plasmids.

Complementation of auxotrophic mutations in T. reesei by heterologous DNA was demonstrated. The plasmid pSal43 containing the argB gene from A. nidulans was used to transform T. reesei argB⁻ mutant strains. The transformants were selected on minimal medium without arginine. The frequency of transformation was around 300 (150 - 400) transformants/µg DNA.

Chromosomal DNA was isolated from the transformants and used for Southern hybridization experiments to verify the proper integration of plasmid DNA into the chromosomal DNA of T. reesei. Multiple tandem copies of pSal43 were integrated in the genome. Southern and dot blot hybridizations showed that in transformants analyzed the copy number of argB varies from appr. 2 to over 100: The ArgB⁺ transformants were shown to be phenotypically 100 % stable through at least 3 generations. This was tested by successive platings of conidia from five transformants onto complete medium and thereafter testing the Arg⁺ phenotype on minimal medium (50-80 colonies/transformant).

### Example 5

### Cotransformation of T. reesei with amdS and argB plasmids.

The possibility to cotransform Trichoderma with a non-selectable plasmid was first shown with the arginine auxotrophic mutant.

The argB⁻ T. reesei strain was transformed with equal molar amounts of A. nidulans plasmids pSaI43 (argB) and p3SR2 (amdS), transformant were selected for Arg⁺ phenotype and tested for acquisition of amdS by streaking on acetamide-CsCl-plates. Of the ArgB transformants , 86 % were also AmdS⁺. Southern analysis of cotransformants indicated that both plasmids were integrated as variable amounts of copies in several different locations in the genome (data not shown).

Double selection a minimal acetamide-CsCI-medium resulted in ~100 big Amd⁺Arg⁺ transformants per µg of DNA, and a number of small colonies, characteristic, of amdS transformation.

When the stability of the ArgB⁺ phenotype of the argB amdS cotransformants was tested, three out of five proved 100 % stable, whereas the other had lost the ArgB⁺ character in 7 % or,80 % of the progeny analysed. The same individual colonies had also lost the AmdS⁺ phenotype. Weather this instability was caused by e.g. the cointegration of argB with the amdS to, the same chromosomal location is not known.

The plasmid pAN5-41B which contains the E. coli lacZ gene coupled in phase to the promoter and N-terminal protein coding region of the A. nidulans glyceraldehydephosphate dehydrogenase gene (gpd) (ref. 55) was also used for cotransformation. In addition, this plasmid contains the A nidulans argB gene and pBR322 sequencies. Prototrophic T. reesei (QM 9414) was cotransformed with the plasmid p3SR2 (amdS) and pAN5-41B, transformants were selected for Amd⁺ phenotype and screened for β-gal expression on acetamide-CsCI-plates containing Xgal. No endogenous T. reesei β-galactosidase activity could be detected when glucose was presend in the medium and pH of the Xgal plates was neutral.

After 1-4 days of growth blue colour was visible. When 1.0/0.7 molar ratio of the plasmids (p3SR2/pAN5-41B) was used in transformation 13 % of the big and 6 % of the small Amd⁺ clones showed β-galactosidase activity, with molar ratio of 1.0/2.6, 39 % and 7 % of Xgal⁺ clones, respectively, were obtained. The presence of both plasmids in Amd⁺ transformants showing blue colour was verified by Southern hybridization (data not shown).

### Example 6

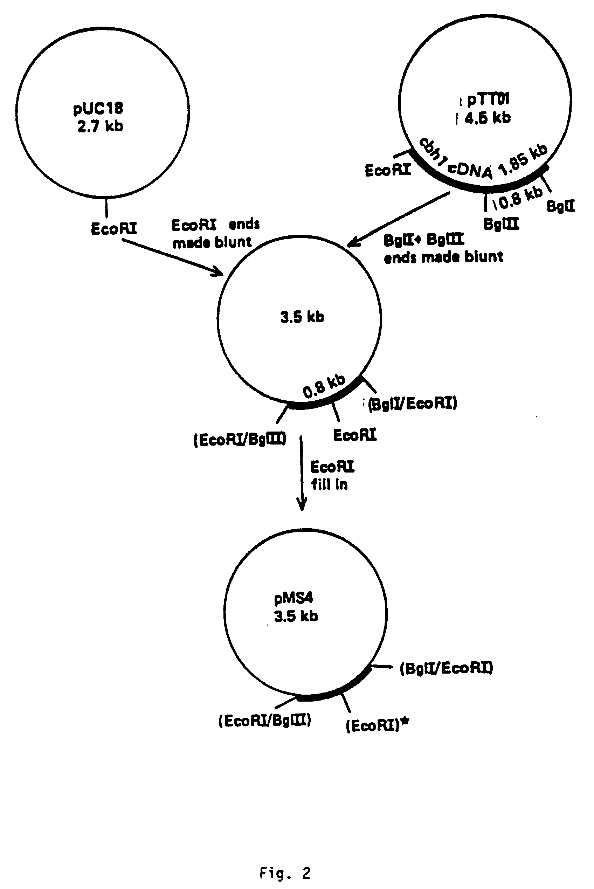

Construction of a cbh1⁻ Trichoderma reesei -strain. The inactivation of different cellulase genes in the manner described allows the construction of a series of T. reesei strains producing a particular combination of cellulases. cbh1⁻ strains are also important for the efficient production of heterologous proteins when using the cbh1 promoter. Trichoderma reesei strain QM 9414 (ATCC 26921) was shown to contain only one chromosomal copy of cbh1 gene by Southern hybridization using cbh1 specific probes and so one recombination event should inactivate the gene. An inactive gene was constructed as follows. The plasmid pTT01 (ref. 28) containing the full length cDNA clone of the cbh1 gene in the vector pUC8 was cut with restriction enzymes Bgl I and Bgl II. The 0.8 kb fragment from the 5' terminal region of the cbh1 gene was isolated from agarose gel by conventional techniques. The fragment was made blunt-ended using S1 nuclease and it was ligated to an Eco RI cut, blunt-ended pUC 18 vector and transformed to E. coil JM 109. DNA from the clone containing the cbh1 gene fragment was isolated and digested with restriction enzyme Eco RI which cuts in the middle of the BgI I - Bgl II fragment of cbh1. The Eco RI -generated termini were filled in and back- ligated. A plasmid pMS4 containing a frameshift mutation in the middle of the truncated cbh1 gene fragment was generated (fig. 2).

Trichoderma was cotransformed with the plasmid pMS4 and the A. nidulans amdS containing plasmid p3SR2 with 3-4 times molar excess of plasmid pMS4.

Transformants were selected on the basis of the amdS⁺ phenotype as described (example 3) and purified on selective medium containing asetamide as a sole carbon source. Purified transformants were grown-on-microtiter plates in 200 µl of Trichoderma minimal medium with 1 % Soika floc cellulose as carbon source and 0.2 % proteose peptone as nitrogen source. The cellulase phenotype was tested by the Ouchterlony immunodiffusion by using undiluted growth media against the CBH I specific antiserum (sheep) as described (ref. 56). A number of transformants were identified which produced normal amount of CBH II but no detectable CBH I.

### Example 7

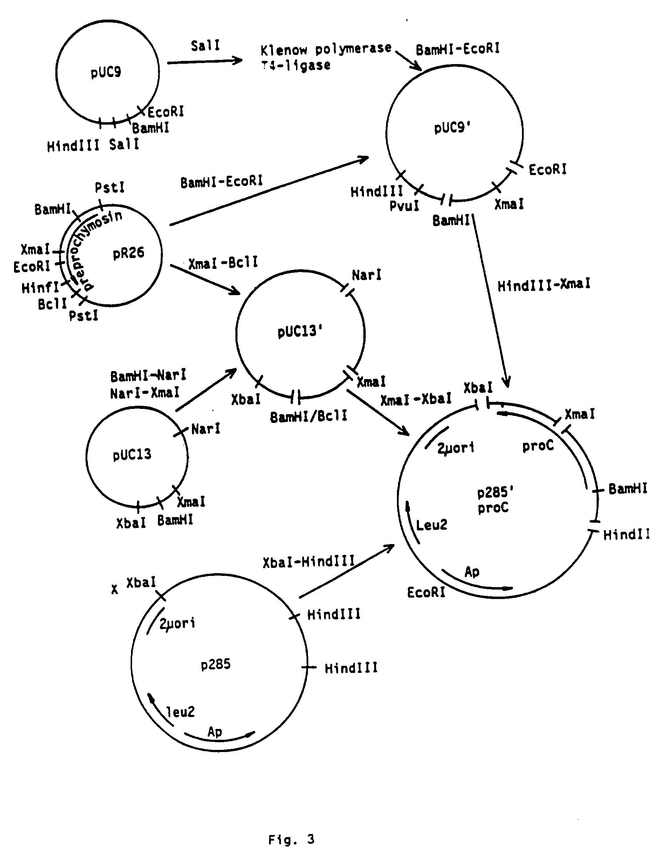

### I. Preparation of Plasmid 285'proC Containing the Prochymosin Gene (fig. 3).

As an example of a heterologous protein in T. reesei we chose to express the prochymosin cDNA (fig. 3). The preprochymosine gene was isolated from a calf stomach cDNA library and inserted into the Pst I site of pBR322 by G-C tailing (refs. 46 and 47) to obtain pR26. pUC9 was cut with SalI and filled out with Klenow polymerase and ligated with T4 ligase. The obtained plasmid was cut with BamHI-EcoRI and the 2.7 kb large fragment was ligated with a 0.47 kb BamHI-EcoRI fragment from pR26 to create pUC9' containing a HindIII site N-terminally of the prochymosin gene. pUC13 was cut with BamHI-NarI and Narl-Xmal and the large respective small fragment was ligated with a 0.64 kb XmaI-BcII fragment of pR26 to obtain plasmid pUC13' containing an Xbal-site C-terminally of the prochymosin gene. A 0.65 kb Xmal-Xbal fragment of pUC13' was ligated with a 0.46 kb Hindlll-Xmal fragment of pUC9' and a 11 kb XbaI-HindIII fragment of p285 to create plasmid p285' proC containing the prochymosin gene as illustrated in fig. 3.

### II. Construction of Plasmid pAMG/Term.

pCAMG91 was digested with Sall and Pstl restriction endonucleases. From such a digests a 698 bp fragment was isolated on an agarose gel. This Sall-Pstl fragment contains the region encoding the 140 bp 3' untranslated part of the glucoamylase mRNA plus 540 bp 3' to the poly(A)-addition site. This 3' fragment was treated with T4-DNA polymerase to "blunt end" the restriction sites before the addition of Xbal linkers and digestion with Xbal restriction enzyme. This 3' end of the glucoamylase gene was ligated to pUC13 linearized with Xbal to create plasmid mAMG/Term containing the glucoamylase gene poly(A) addition region.

### III. Construction of Plasmid pMT837

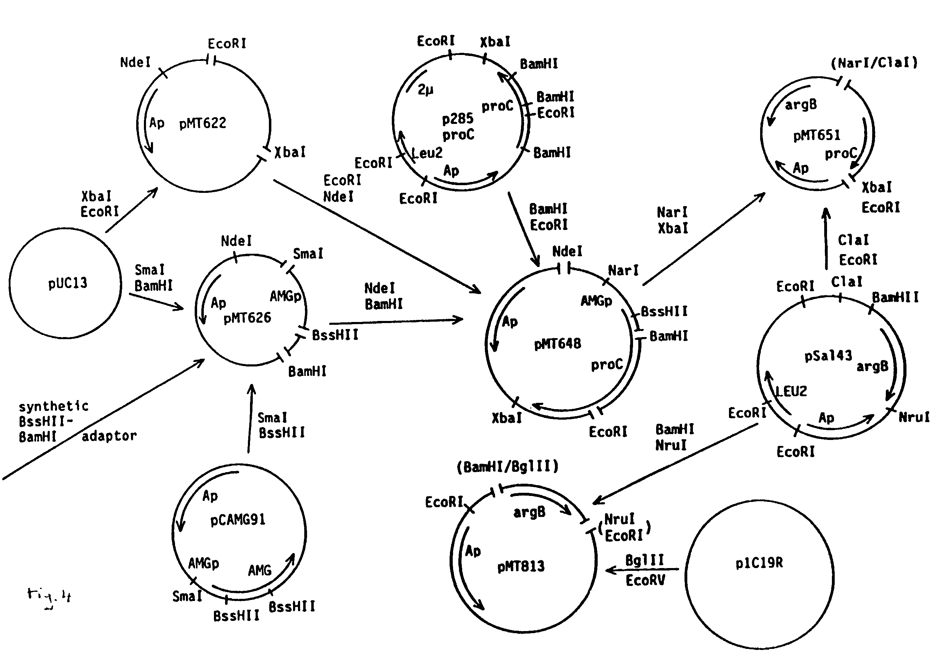

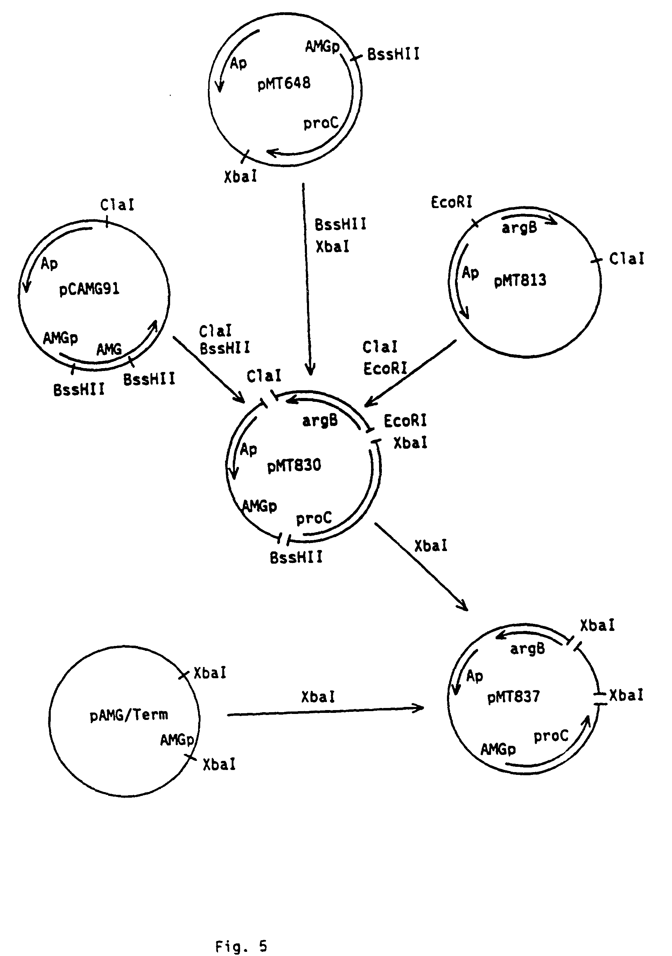

A 0.5 kb SmaII-BssHII fragment of pCAMG91 containing the promoter and the sequences coding for the glucoamylase (AMG) signal and leader peptide was ligated to SmaI-BamHI digested pUC13 and a synthetic BssHII-BamHI adaptor encoding the first 6 amino acids of prochymosin. From the resulting plasmid, pMT626, a 0.8 kb NdeI-BamHI fragment was isolated and ligated to a 0.5 kb BamHI-EcoRI fragment from p285'proC containing the sequence for the N-terminal half of proC and to a 3.0 kb EcoRI-Ndel fragment of pMT622 containing the C-terminal part of the sequence for proC. (pMT622 is simply a EcoRI-XbaI subclone of p285' proC in pUC13). The resulting plasmid pMT648 (see fig. 4) contains the entire prochymosin gene proceeded by AMG promoter end signal/Ieader encoding sequences. pMT648 was further modified to contain the argB gene of A. nidulans (ref. 48). A 1.8 kb Narl-filled in Xbal fragment of pMT648 was ligated to a 6.8 kb Clal filled-in EcoRI fragment of pSaI43, to give pMT651. Sequences further upstream of the AMG promoter (upstream activating sequences; UAS's) were furthermore inserted. This was achieved by ligating a 3.7 kb ClaI-BssHII fragment from the original clone pCAMG91 to the 1.1 kb BssHII filled in Xbal fragment of pMT648 containing the proC sequences, and the argB gene as a 3.1 kb Clal-filled In EcoRI fragment from pMT813 (pMT813 is the argB gene cloned as a 3.1 kb BamHI-Nrul fragment cloned into EcoRV-BgIII cut piC19R). The resulting plasmid, pMT830, has got an expression unit containing the UAS's, promoter, signal and leader sequences from AMG and the entire gene for proC. Finally the terminator sequence of AMG is taken as a 0.6 kb XbaI-XbaI fragment from plasmid pAMG/Term and inserted into the Xbal cut and dephosphorylated pMT830 to give pMT837. The construction of pMT837 is illustrated in fig. 5.

### Example 8

### Production of chymosin using pMT837.

T. reesei strains QM 9414 and RUT-C-30 were cotransformed with plasmids pMT837 and p3SR2. Plasmid pMT837 contains the prochymosin gene proceeded by the AMG promoter and signal/leader sequence. The construction of plasmid pMT837 was described in Example 7 (see also figs. 3-5). Cotransformation and selection was carried out as in Example 5.

For chymosin production transformants were cultured on minimal medium containing 1 % Solka floc cellulose and 0.2 % proteose peptone.

The mycelia were collected and the supernatant was concentrated, when necessary, by TCA precipitation and diluted into 2 M NaOH 10 mM Tris (pH 8.5). The samples were fractioned on SDS-page (7.5 % - 15 % polyacrylamide gradient) and electroblotted to a nitrocellulose filter. The filters were incubated with rabbit prochymosin antiserum and stained with 4-chloro-1-naftol using α-rabbit-IgG-peroxidase conjugate purchased from Sigma.

Chymosin was shown to be secreted into the medium approximately at the level of 1 µg/l. Since the AMG promoter clearly functions inefficiently in T. reesei homologous T. reesei promoter-terminator vectors were constructed to improve the production level of chymosin.

### Example 9

The construction of heterologous expression vectors for production of calf prochymosin in T. reesei using cbh1 promoter.

### I. The joining of prochymosin gene to cbh1 terminator region.

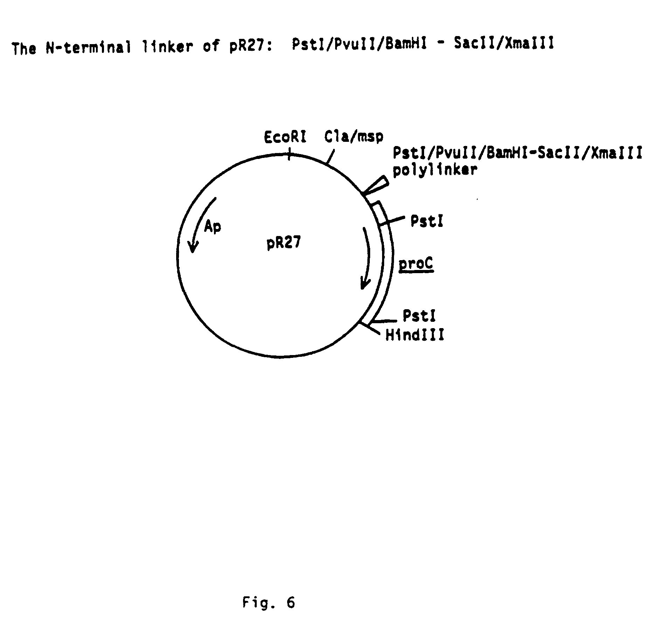

The calf prochymosin gene was obtained from plasmid pR27 (fig. 6). The Pstl - Pstl fragment (~ 1110 bp) containing almost the whole gene was isolated from agarose gel by conventional techniques and ligated to the Pst1 site of pUC 19.

This plasmid was partially digested with Pstl, ends made blunt with S1 nuclease and an Avall terminator fragment ( - 750 bp) of cbh1 gene (blunt ends with Klenow fragment) was ligated into this plasmid. The terminator region of cbh1 gene was obtained from the terminal 1.8 kb BamHI fragment of cbh1 cDNA subcloned in pBR322 (ref. 24).

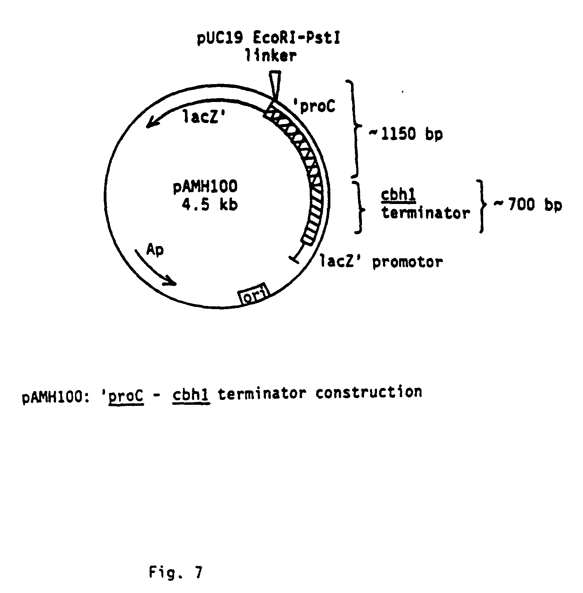

This plasmid containing the 'pro C fragment coupled with the terminator region of cbh1 gene in pUC19 was called pAMH100 (fig. 7).

### II. The fusion of cbh1 promoter region and prochymosin coding region using BgII-SacII adaptor.

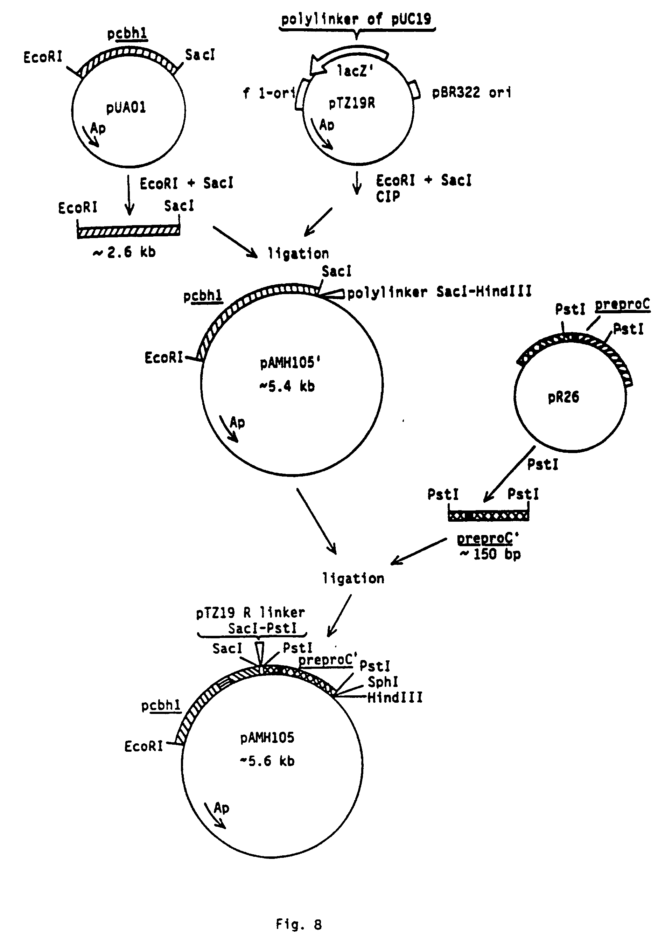

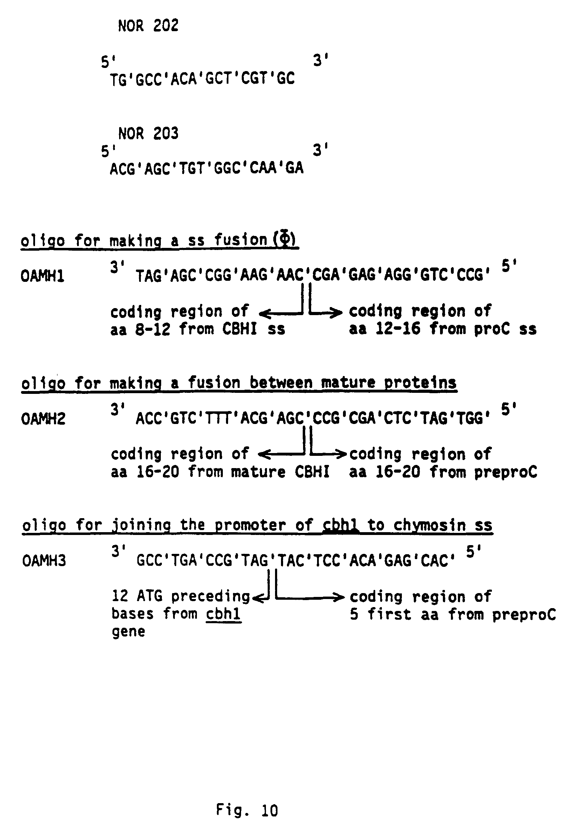

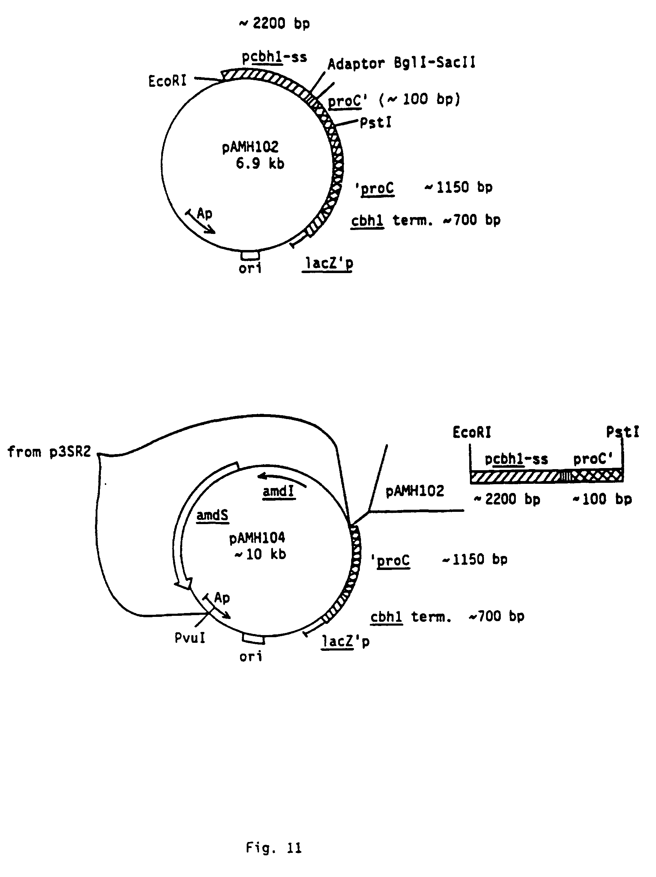

The SacII - Pstl fragment ( 80 bp) coding for the N-terminal region of prochymosin was isolated from plasmid pR27 (see fig. 6). The cbh1 promoter region was first subcloned from the genomic clone λ 44A as a 2.8 kb long EcoRI - EcoRI fragment into pUC18 (plasmid pUA01, fig. 8), and then a ~ 2.2 kb long EcoRI - Bgll fragment was isolated from this subclone. The Bgl I site is located in the middle of the signal sequence of cbh1 gene. The precise joining of the ~ 2.2 kb long EcoRI - Bgll fragment, containing the promoter and about half of the signal peptide coding region of cbh1 gene, to the ~ 80 bp SacII - Pstl prochymosin fragment is mediated by the Bgll - SacII adaptor (NOR 202 + NOR 203, fig. 10). These fragments together with the adaptor were ligated into pUC19 digested with EcoRI - Pstl. This plasmid codes for a complete signal sequence of CBH I fused to the first amino acid of prochymosin followed by some of its N-terminal sequences. Finally, from this construction the Eco RI pcbh1 ss - proC - PstI fragment was isolated, ligated from its 3' end into pAMH100 digested with Sal I and Pst I. The 5' end of the fragment end 3' end of plasmid pAMH100 was filled in with Klenow and ligated. Thus the promoter of cbh1 gene and proC' fragment was transferred in front of 'proC followed by the cbh1 terminator area. The construction was named pAMH102 (Fig. 11).

### III. The addition of a selectable marker to chymosin expression plasmid.

As a selectable marker in the expression plasmids either the amdS gene of A. nidulans (refs. 33, 41) or the argB gene of A. nidulans can be used (ref. 42)).

The amdS gene was isolated as a Pvu I - Sal I fragment from p3SR2 and ligated to the 6 kb long Pvul - Sall fragment of pAMH102. This selectable vector was named pAMH104 (Fig. 11).

### IV. The precise fusion of the cbh1 promoter and preprochymosin coding sequences using oligonucleotides

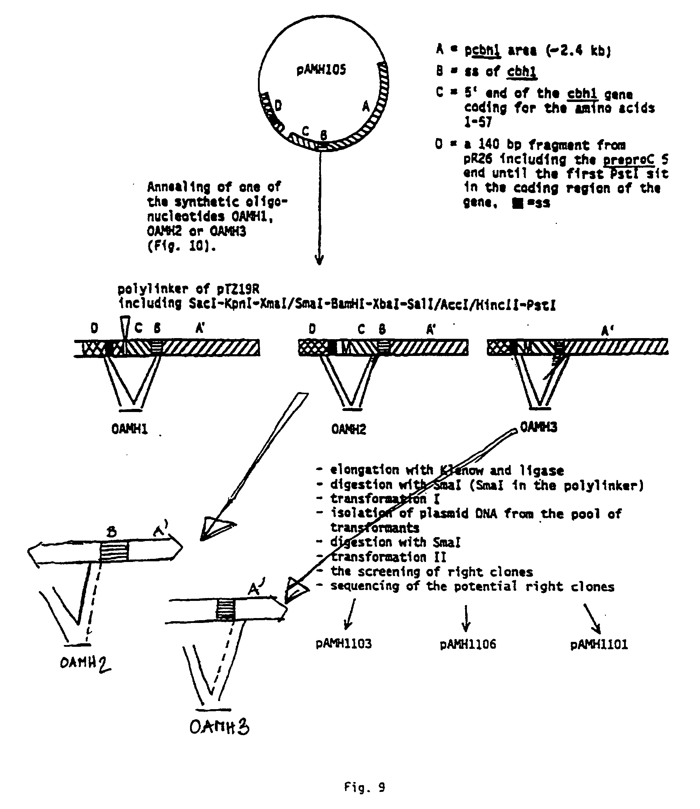

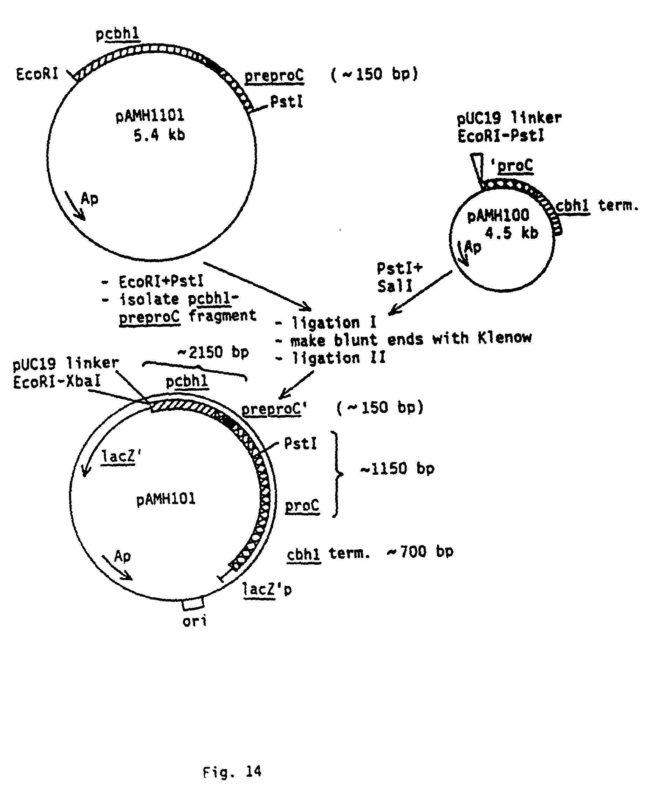

The aminoterminal Pst1 fragment (~ 150 bp) of preprochymosin was isolated from pR26 (fig. 3) which includes a complete preprochymosin cDNA clone, starting 12 bp upstream from ATG, inserted into Pstl site in pBR 322. This fragment was subcloned into the polylinker of pTZ19R together with the cbh1 Eco RI - Sac I (~ 2.6 kb) promoter fragment which also includes the signal sequence coding region of cbh1 (fig. 8). The cbh1 Eco RI - Sac I fragment is obtained from a 2.8 kb Eco RI - Eco RI subclone in pUC 18 (pUA01, fig. 8) of the original λ44A clone (ref. 24). pTZ19R (Pharmacia) i a pUC19 based plasmid including the F1 origin of replication and the T7 promoter enabling the use of ss-template (single stranded) for oligonucleotide mutagenesis. The construction of the resulting plasmid (pAMH 105) is illustrated in Figure 8. From this plasmid the sequences between cbh1 promoter area and proC ATG were deleted by loop-mutagenesis using a specific oligonucleotide, OAMH 3 (fig. 10). The performance of oligonucleotide directed mutagenesis is illustrated in figure 9. The oligonucleotide in question was phosphorylated, annealed to ss pAMH 105 DNA (ref. 57, ss-DNA was isolated from JM103/pAMH105 as described by Pharmacia) in molar ratio 20:1, respectively. The oligonucleotide primer was elongated using Klenow polymerase and T4 ligase as described in ref.57. The elongated mixture was digested with Sma I (resides in the polylinker of pTZ19R, see fig. 9) prior to transformation into the mutL mismatch repair deficient strain of E. coli, BMH71.18 (ref.58). The pool of transformants was grown overnight In 5 ml liquid culture (Luria broth as described In ref. 59, with 100 µg/ml ampicillin). Plasmid DNA was isolated from pool of transformants and it was redigested with Smal and retransformed Into E. coli JM109 strain. The screening of potential deletion clones was performed by digestion using different restriction enzymes and the specificity of deletion was further confirmed by sequencing. The resulting plasmid was called pAM1101 (fig. 14). This plasmid was further digested with EcoRI and Pstl and the resulting pcbhl-preproC fragment was isolated (fig. 14) and ligated at its Pstl-end to pAMH100 plasmid digested with PstI and SaII. The ends of the resulting ligated fragment were made blunt by Klenow and ligated. The resulting plasmid was called pAMH101 and it contains the cbh1 promoter area fused to the signal sequence of prochymosin and the whole prochymosin coding region fused to the terminator area of cbh1 gene (fig. 14).

### V. The precise fusion of the cbh1 promoter and prepro gene by signal sequence fusion performed by using oligonucleotides.

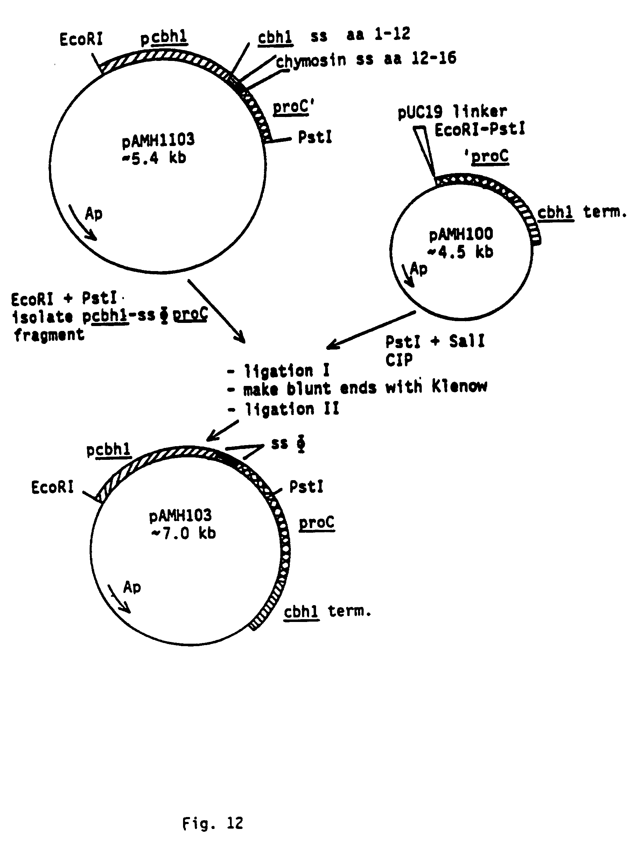

The construction of plasmid pAMH1103 (fig. 12) was performed essentially as pAMH1101 described in details in the preceding chapter IV with the exception that the specific oligonucleotide used for this construction was OAMH1 (fig. 10 and 9). The resulting plasmid pAMH1103 contains a signal sequence fusion including amino acids (aa) 1-12 from cbh1 signal sequence fused to aa 12-16 from preproC signal sequence preceding the aminoterminal part (~ 140 bp) of the coding region of proC gene (fig. 12). From plasmid pAMH1103 the pcbh1-sso-proC' (o = fusion) fragment was subcloned into pAMH100 essentially as described in the preceding chapter IV (see Fig. 12). Thee resulting plasmid pAMH 103 includes the promoter area of cbh1 and signal sequence fusion of cbh1 and preproC preceding the proC coding region fused to the cbh1 terminator area.

### VI The precise fusion of the cbh1 coding region to proC coding region by using oligonucleotides.

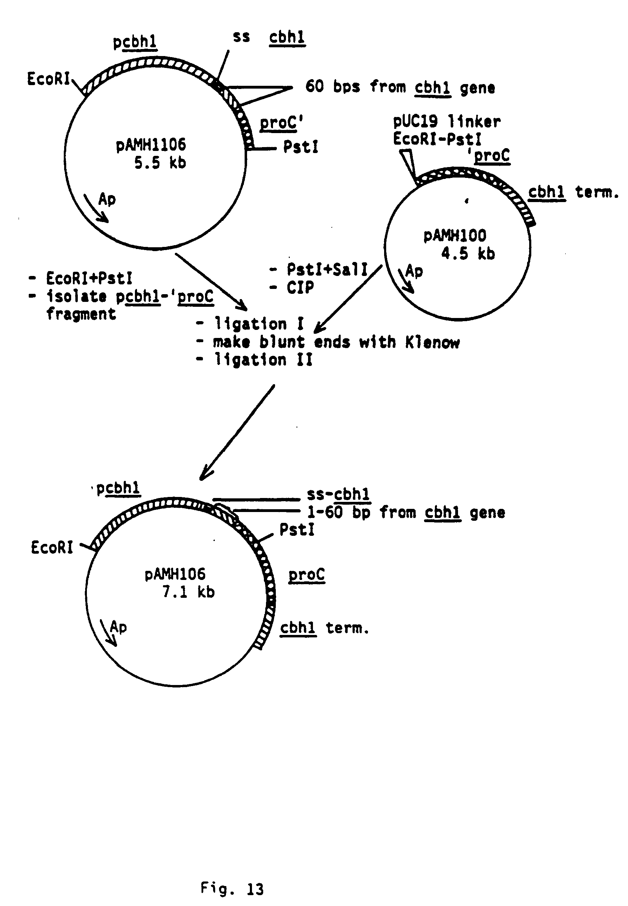

The construction of plasmid pAMH1106 (fig. 13) was performed essentially as pAMH1101 described in details in the preceding chapter IV with the exception that the specific oligonucleotide used for this construction was OAMH2 (fig. 10 and 9). From the resulting plasmid pAMH 1106 including the promoter area of cbh1, signal sequence of cbh1 and the coding region of 1-20 first aa of mature CBH I fused to the coding region of proC the fragment containing pcbh1-mature o-proC' was subcloned into pAMH100 essentially as described in the preceding chapter IV (Fig. 12). The resulting plasmid pAMH106 includes the promoter area of cbh1, signal sequence of cbh1. coding region of aa 1-20 of mature CBHI fused to the coding region of proC.

### Example 10

### Production of chymosin using pAMH102 and pAMH 104.

T. reesei strains QM 9414 and RUT-C-30 were cotransformed with plasmids pAMH102 and p3SR2 in molar ratio 5:1, respectively. The construction of plasmid pAMH102 was described in Example 9 chapter II. Cotransformation and selection was carried out as in Example 5. The transformants were purified on selective asetamide plates as described in Example 3.

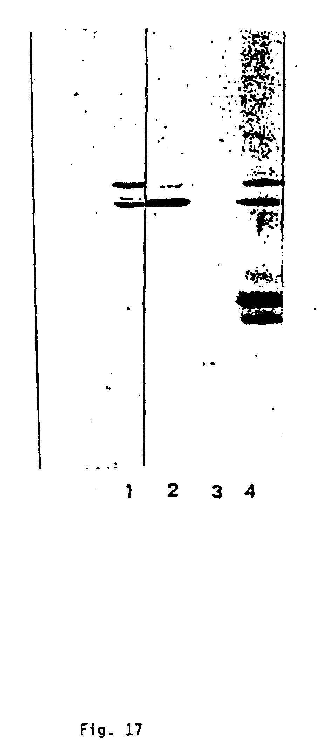

For chymosin production transformants were cultured on minimal medium (10 to 50 ml) containing 1 % Solka floc cellulose and 0.2 % proteose peptone. The mycelia were collected and the supernatant was concentrated by TCA precipitation and diluted into 2M NaOH 10 mM Tris (pH 8.5). The mycelia were broken in the presence of liquid nitrogen, broken cells were pelleted and the supernatant treated in the same way as the culture media. The samples were fractioned on SDS-page (7.5 % - 15 % polyacrylamide gradient) and electroblotted to a nitrocellulose filter. The filters were incubated with rabbit prochylisin antiserum and α-rabbit-IgG-AP (alkaline phosphatase) conjugate and stained with nitro blue tetrazolium and 5-brome-4-chloro-3-indolyl phosphate purchased from Promega Biotec. The amount of chymosin inside and outside the mycelium was compared (fig. 17). It was shown by Southern hybridization that the clones containing higher number of copies of plasmid pAMH102 integrated into the fungal chromosomal DNA also produced more chymosin. When using a one copy transformant the amount of secreted chymosin was 100 µg/l of culture medium when approximated from Western gels (fig. 17). The secreted prochymosin was shown to be processed to an active chymosin by Western gels and by the milk clotting activity (chymosin clots milk by cleavage of β-kasein determination (ref. 60). The amount of various forms of chymosin (preproC, proC, C and chymosin derived degradation products inside the cell) inside the mycelium was determined to be 0.04 % of mycelial total protein (fig. 17) and the amount of secreted chymosin was 60 % of the total chymosin produced. This shows the efficiency of T. reesei to secrete even heterologous gene products outside the mycelia.

Transformants with several copies of plasmid pAMH104 (fig. 11), able to secrete larger amounts of prochymosin were screened for by determining the milk clotting activity of the growth media. The best transformant out of the 40 studied was found to secrete 500 µg/I of culture medium as determined on Western gels and by milk clotting activity.

Since the amount of secreted, active chymosin was increased 200-fold when cultures were grown in a 5 I laboratory fermenter compared to the small-scale cultures (10-50 ml), the amount of chymosin produced by this type of strain is around 100 mg/l.

### Example 11

### Construction of a general expression vector for production of homologous and heterologous proteins in T. reesei.

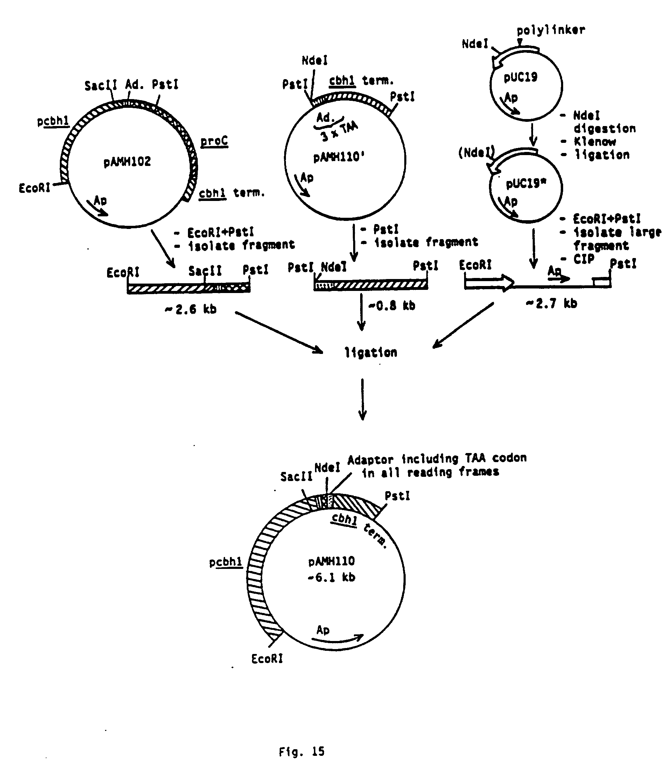

In order to be able to construct vectors for production of various proteins in T. reesei a general expression vector including the promoter and terminator areas of cbh1 gene was constructed. The cbh1 terminator was subcloned together with an adaptor including the STOP codon TAA in three reading frames into Pst I site of pUC19 resulting in plasmid pAMH110' (fig. 15). The Pst I terminator fragment was isolated from pAMH110', the cbh1 promoter area was isolated from pAMH102 as an Eco RI - Pst I fragment including the aminoterminus of proC gene and these fragments were subcloned into an Eco RI - Pst I digested pUC19* from which the single Nde I site had been made blunt with Klenow prior to this subcloning step. The resulting plasmid pAMH110 includes the promoter and terminator of cbh1 gene and between these sequences a stuffer fragment which can be removed by digestion with Sac II and Nde I. After the ends are made blunt any cDNAs or chromosomal copies of genes can be inserted between the promoter and terminator (fig. 15).

### Example 12

### The expression of T. reesei endoglucanase I under cbh1 promoter.

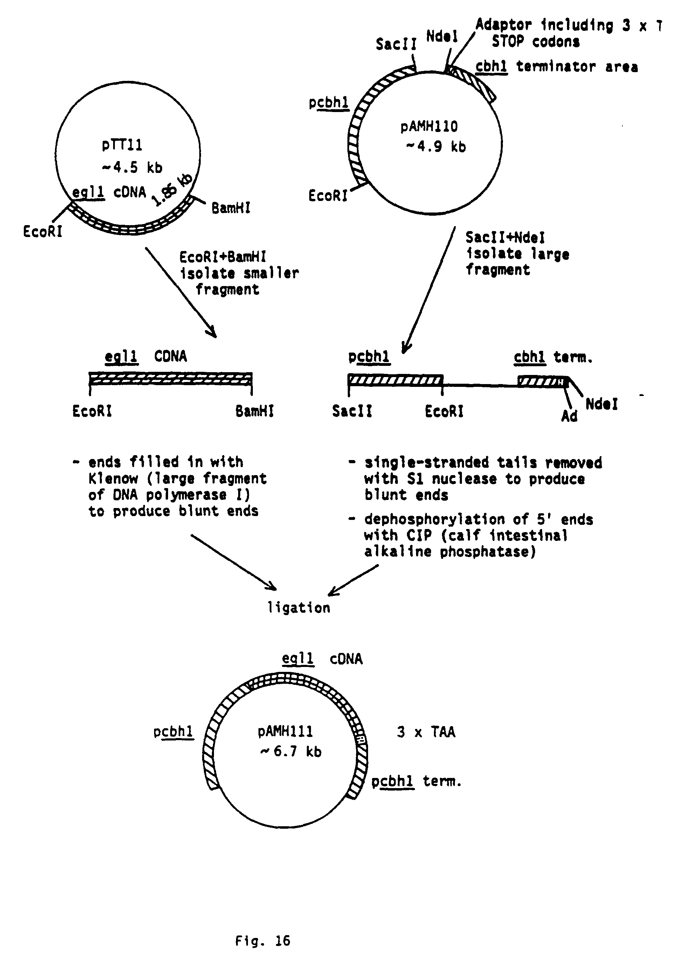

In order to increase the amount of endoglucanase produced, the egl1 gene was linked to the more powerfull cbh1 promoter. The cDNA for T. reesei endoglucanase I was subcloned as an Eco RI - Bam HI fragment into general expression vector pAMH110 (described in example 11) which was first digested with Sac II - Nde I to delete the stuffer fragment (fig. 16). The resulting plasmid pAMH111 including egl1 gene between the promoter and terminator of cbh1 gene was cotransformed with p3SR2 to T. reesei QM 9414 (ATCC 26921) in molar ratio 5:1, respectively. The transformants were selected for AmdS⁺ phenotype and further purified on selective medium. Six individual transforments were grown for 4 days in cellulase inducing medium (Solke floc as a carbon source, 1 %) in 50 ml liquid cultures. The culture supernatants were then tested for endoglucanase activity by measuring the release of reducing sugars from hydroxyethylcellulose (0.1 %, ref. 12). The EG I activities of transformants were compared to a control (QM 9414) end the best transformant was shown to secrete 4 times the endoglucanase activity of the control strain. This example shows that it is possible to modify the amounts of different cellulolytic enzymes in T. reesei by changing the respective promoter.

### REFERENCES

1. Simmons, E.G., Classification of some cellulase producing Trichoderma species. In: Abstracts of Second International Mycological Congress. Tampa, Florida, USA. H.E. Aigelow & E.G. Simmons (Eds.) 1977, p. 618.
**2.** Berghem, L.E.R. & Pettersson, L.G., The mechanism of enzymatic cellulose degradation. Purification of cellulolytic enzyme from Trichoderma viride active on highly ordered cellulose. Eur.J.Biochem. 97 - (1973), 21-30.
**3.** Gong, C.S., Ladisch, M.R. & Tsao, G.T., Biosynthesis, purification and mode of action of cellulases of Trichoderma reesei. Adv.Chem.Ser. 181 (1979), 261-287.
**4.** Fägerstam, L.G. & Pettersson. L.G., The 1,4-β-glucan celloblohydrolases of Trichoderma reesei QM 9414. A new type of synergism FEBS Letters 119 (1980), 97-100.
**5.** Enari, T.-M., Microbial Cellulases. In: Microbial Enzymes and Biotechnology. W.M. Fogarty (Ed.). Applied Science Publishers, London and New York, 1983, pp. 183-223.
6. Gilbert, I.G. & Tsao, G.T., Physical properties of T. viride cellulase. Ann. Reports on Fermentation Process 6 (1983), 323-358.
7. Shoemaker, S.P. & Brown, Jr., R.D., Enzymic activities of endo-1,4-β-glucanases purified from Trichoderma viride. Biochim.Biophys. Acta 523 (1978), 133-146.
8. Shoemaker S.P. & Brown, Jr., R.D., Characterization of endo1,4-β-glucanases purified from Trichoderma viride. Biochim.Biophys.Acts 523 (1978), 147-161.
9. Bissett, F.H., Analysis of cellulase proteins by high performance liquid chromatography. J.Chromatog. 178 (1979), 517-523.
10. Farkas, V.A., Jalanko, A. & Kolarova, N., Characterization of cellulase complexes from Trichoderma reesei QM 9414 and its mutants by means of analytical isoelectrofocusing in polyacrylamide gels. Biochem.Biophys.Acta 706 (1982), 105-110.
11. Enari, T.-M., Niku-Paavola, M.-L. & Nummi, M., Comparison of cellulolytic enzymes from Trichoderma reesei and Aspergillus niger. In: Proceedings of 2nd International Symposium on Bioconversion and Biochemical Engineering. T.K. Ghose (Ed.). Indian Institute of Technology, New Delhi 1 (1980), 87-95.
12. Bailey, M.J. & Nevalainen, K.M.H., Induction, isolation and testing of stable Trichoderma reesei mutants with improved production of solubilizing cellulase. Enzyme Microb.Technol. 3 (1981), 153-157.
13. Andreotti, R., Medeiros, J., Roche, C. & Mandels, M., Effects of strain and substrate on production of cellulases by Trichoderma reesei mutants. In: Proceedings of 2nd International Symposium on Bioconversion and Biochemical Engineering. T.K. Ghose (Ed.) Indian Institute of Technology, New Delhi 1 - (1980), 353-388.
14. Farkas, V., Labudova, I., Bauer, S. & Ferenczy, L., Preparation of mutants of Trichoderma viride with increased production of cellulase. Folia Microbiol. 26 (1981), 129-132.
15. Montenecourt, B.S. & Eveleigh, D.E., Selective screening for the isolation of high yielding cellulase mutants of T. reesei. Adv.Chem.Ser. 181 (1979), 289-301.
16. Mandels, M., Weber, J. & Parizek, R., Enhanced cellulase production by a mutant of Trichoderma viride, Appl.Microbiol. 21 (1971), 152-154.
17. Gallo, B.J., Andreotti, R., Roche, C., Ruy, D. & Mandels, M., Cellulase production by a new mutant strain of Trichoderma reesei MCG 77. Biotechnol.Bioengineer. Symp. 8 (1979), 89-102.
18. Warzywoda, M., Vandecasteele, J.P. & Pourquie, J., A comparison of genetically improved strains of the cellulolytic fungus Trichoderma reesei. Biotechnol.Lett. 5 (1983), 243-246.
19. Montenecourt, B.S. & Eveleigh, D.E., Preparation of mutants of Trichoderma reesei with enhanced cellulose production. Appl.Environ. Microbiol. 34 (1977), 777-782.
20. Sheir-Neiss, G. & Montenecourt, B.S., Characterization of the secreted celluloses of Trichoderma reesei wild type and mutants during controlled fermentations. App.Microbiol.Biotechnol. 20 (1984), 46-53.
21. Shoemaker, S.P., Raymond, J.C. & Bruner, R., Celluloses: Diversity amongst improved Trichoderma strains. In: Trends in the Biology of Fermantotions for Fuels and Chemicals A.E. Hollaender (Ed.). Plenum Press. (1981), 89-109.
22. Nevalainen, K.M.H., Palva, E.T. & Bailey, M.J.B., A high cellulose-producing mutant strain of Trichoderma reesei. Enzyme Microb.Technol. 3 (1980), 59-60.
23. Shoemaker, S., Schweickart, V., Ladner, M., Gelfand, D., Kwok, S., Myambo, K. & Innis, M., Molecular cloning of exocellobiohydrolase I derived from Trichoderma reesei strain L27. BIO/TECHNOLOGY 1 (1983), 691-696.
24.Teeri, T., Salovuori, I. & Knowles, J., The molecular cloning of the major cellulose gene from Trichoderma reesei BIO/TECHNOLOGY 1 (1983), 696-699.
25. Penttilä, M., Lehtovaara, P., Nevalainen, H., Bhikhabhai, R. & Knowles, J. 1986. Homology between cellulose genes of Trichoderma reesei: complete sequence of the endoglucanase I gene.Gene (1986) 45: 253-263.
26. EP-A-0 137. 280 (Cetus Corporation).
27. Van Arsdel, J.N.V., Kwok, S., Schweickart, V.L., Ladner, M.B., Gelfand, T.H. & Innis, M.A., Cloning, characterization and expression in Saccharomyces cerevisiae of endoglucanase I from Trichoderma reesei. BIO/TECHNOLOGY 5: 60-64.
28. WO-A-85/04672 (Alko Ltd)
29. Chen, C.M., Gritzali, M. & Stafford, T.W., Nucleotide sequence and deduced primary structure of cellobiohydrolase II from Trichoderma reesei. BIO/TECHNOLOGY (1987) 5: 274-278.
30. Saloheimo, M., Lehtovaara, P., Penttilä, M., Teeri, T.T., Stählberg, J., Johansson, G., Pettersson, G., Claeyssena, M., Tomme, P. & Knowles, J., A new endoglucanase from Trichoderme reesei: Characterization of both gene end enzyme. Submitted for publication in BIO/TECHNOLOGY.
31. Case, M., Schweizer, M., Kushner, S.R. & Giles, N.H., Efficient transformation of Neurospora crassa by utilizing hybrid plasmid DNA. Proc.Natl.Aced.Sci. USA 76 (1979), 5259-5263.
32. Ballance, J., Buxton, F.P. & Turner, G., Transformation of Aspergillus nidulans by the orotidine-5'-phosphate decarboxylase gene of Neurospora crassa. Biochem.Biophys.Res.Commun. 112 (1983), 284-289.
33. Tilburn, J., Schazzocchio, C., Taylor, G.T., Zabickyzissman, J.H. Lockington, R.A. & Davies, R.W., Transformation by integration in Aspergillus nidulans. Gene 26 (1983), 205-221.
34. Yelton, M., Hamer, J.E. & Timberlake, W.E., Transformation of Aspergillus nidulans by using a trpC plasmid. Proc.Natl.Acad.Sci. USA 81 (1984), 1470-1474.
35. Kelly, J.M. & Hynes, M.J., Transformation of Aspergillus niger by the amdS gene of Aspergillus nidulans EMBO Journal 4 (1985), 475-479.
36. Nevalainen, H., Genetic improvement of enzyme production in industrially important fungal strains. Technical Research Center of Finland, Publications 26 (1985).
37. Beckwith, J.R., Pardee, A.B., Austrian, R. & Jacob, F., Coordination of the synthesis of the enzymes in the pyridimide pathway of E. coli. J.Mol.Biol. 5 (1962), 618-634.
38. Ballance, D.J. & Turner, G., Development of a high-frequency transforming vector for Aspergillus nidulans. Gene 36 (1985), 321-331.
39. Creighton, T.E., N-(5'-Phosphoribosyl) anthranilate isomerase-indol-3-ylglycerol phosphate synthetase of tryptophan biosynthesis. Biochem.J. 120 (1970), 699-707.
40. Keesey, J.K.J.R. & DerMoss, J.A., Cloning of the trpl gene from Neurospora crassa by complementation of a trpC mutation in Escherichia coli. J.Bacteriol. 152 (1982), 954-958.
41. Hynes M.J., Corrick, C.M. & King, J.A. Isolation of genomic clones containing the amdS gene of Aspergillus nidulans and their use in the analysis of structural and regulatory mutations. Mol.Cell.Biol. 3 - (1983), 1430-1439.
42. Casadaban, M.J., Martinez-Arias, A., Shapira, S.K. & Chon, J., β-galactosidase gene fusions for analyzing gene expression in Escherichia coli and yeast. Methods Enzymol. 100B (1983), 293-308.
43. Raeder, U. & Broda, P., Rapid preparation of DNA from filamentous fungi. Lett. in Appl.Microbiol. 1 - (1983) 17-20.
44. Sollazzo, M., Frank, R. & Cesareni, G., High-level expression of RNAs and proteins: the use of oligonucleotides for the precise fusion of coding-to-regulatory sequencs. Gene 37 (1985), 199-206.
45. John, M.A. & Peberdy, J.F. Transformation of Aspergillus nidulans using the argB gene. Ensyme Microb.Technol. 6 (1984), 386-389.
46. Chirgwin, J.M., Przybyla, A.E., MacDonald, R.J. & Rutter, W.J., Isolation of biologically active ribonucleic acid from sources enriched in ribonuclease. Biochemistry 18 (1978), 5294-5299.
47. Truelsen, E:, Gausing, K., Jochimsen, B., Jørgensen, P. & Marcker, K.A., Cloning of soybean leghemoglobin structural gene sequences synthesized in vitro. Nucleic Acids Res. 6 (1979), 3061-3072.
48. Berse, B., Dmochowska, A., Skrzypek, M. Wegleriski, P., Bates, M.A. & Weiss, R.L. Cloning and characterization of the ornithine carbamoyltransferase gene from Aspergillus nidulans. Gene 25 (1983), 109-117.
49. Fincham, J.R.S. et al., Fungal genetics, Botanical Monographs vol. 4, Blackwell Scientific Publications Edinburgh, p. 639, Fourth edition, 1979.
50. Doi, Y., Revision of the Hypocreales with cultural observations. II. Hypocrea dichromospora sp. nov. and its Trichoderma state. Bull.Nat.Sci.Mus. Tokyo 11 (1968), 185-189.
51. Yanish-Perron, C., Vieira, J. & Messing, J., Improved MJ13 phage cloning vectors and host strains: nucleotide sequences of the M13 mp 18 and pUC 19 vectors. Gene 33 (1985), 103-119.
52. Boel, E., Hansen, M.T., Hjort, I., Høegh, I. & Fiil, N.P., Two different types of intervening sequences in the glucoamylase gene from Aspergillus niger. The EMBO Journal 3 (1984), 1581-1585.
53. Marsh, J.L., Erfle, M. & Wykes, E.J. The pIC plasmid and phage vectors with verstile cloning sites for recombinant selection by insertional inactivation. Gene 32 (1984), 481-485.
**54.** Vieira, J. & Messing, J., The pUC plasmids, an M13mp7 - derived system for insertion mutagenesis and sequencing with synthetic universal primers. Gene 19 (1982), 259-268.
**55.** Van Gorcom, R.F.M., Punt, P.J., Pouwels, P.H., & Van den Hondel, C.A.M.J.J. A system for the analysis of expression signals in Aspergillus. Gene 48 (1986), 211-217.
**56.** Nummi, M., Niku-Paavola, M.-L., Lappalainen, A., Enari, T.-M. & Raunio, V., Cellobiohydrolase from Trichoderma reesei. Biochem.J. 215 (1983), 677-683.
**57.** Eghtedarzadch, M.K. & Henikoff, S., Use of oligonucleotides to generate large deletions. Nucl. Acids Res. 14 (1986), 5115.
58. Obtained from Paul Thomas, Imperial College, Dept. of Chemistry, London.
59. Miller, J.H., Experiments in molecular genetics (1972), Cold Spring Harbor Laboratory, Cold Spring Harbor, New York.
60. The method for determining the activity of chymosin in cleavage of milk β-kasein has been described by Siika-aho, M. (1983) Mikrobirenniinin tuottaminen, MSc Thesis. Biotechnical Laboratory, VTT, Tietotie 2, 02150 Espoo, Finland.
61. EP-A- 0215594 (Genencor, Inc)

The features disclosed in the foregoing description, in the following claims and/or in the accompanying drawings may, both separately and in any combination thereof, be material for realising the invention in diverse forms thereof.

## Claims

1. A Trichoderma strain stably transformed with a vector system comprising:
a) a gene encoding a desired protein product,
b) functions facilitating gene expression including DNA sequences comprising promoters/enhancers operably linked to said gene to control expression of the desired product, and
c) a selection marker capable of selecting stable Trichoderma transformants, the selection marker not being derived from the Neurospora crassa pyr-4 gene.

2. A strain according to Claim 1, wherein the vector system further comprises a signal/leader sequence fused upstream to the 5' end of the gene for the desired protein product.

3. A strain according to Claim 1 or 2, wherein the promoter of the vector system can function in Trichoderma.

4. A strain according to Claim 1, wherein the promoter of the vector system is derived from a gene for protein heterologous to Trichoderma.

5. A strain according to Claim 2, wherein the signal/leader sequence of the vector system is derived from a gene for a protein secreted by Trichoderma.

6. A strain according to Claim 2, wherein the signal/leader sequence of the vector system is derived from a secreted protein heterologous to Trichoderma.

7. A strain according to Claim 5 to 6, wherein the signal/leader sequence of the vector system is derived from the signal/leader sequence of the desired proteins.

8. A strain according to Claim 5, wherein the signal sequence of the vector system is the Trichoderma reesei cbh1 signal sequence.

9. A strain according to Claim 6, wherein the signal sequence of the vector system is the Aspergillus glucoamylase signal sequence.

10. A strain according to Claim 1, wherein the desired proteins is homologous to Trichoderma.

11. A strain according to Claim 1, wherein the desired protein is heterologous to Trichoderma.

12. A strain according to Claim 11, wherein the desired product is prochymosin.

13. A strain according to Claim 10, wherein the desired protein is T. reesei endoglucanase (EG I).

14. A strain according to Claim 3, wherein the promoter of the vector system is the Trichoderma reesei chb1 promoter.

15. A strain according to Claim 4, wherein the promoter of the vector system is the Asperillus glucoamylase promoter.

16. A strain according to Claim 1, wherein the selection marker of the vector system is derived from the Aspergillus nidulans amdS gene, the Aspergillus nidulans or Trichoderma reesei argB gene, the Trichoderma reesei pyr4 gene or Aspergillus nidulans trpC gene.

17. A strain according to Claim 1, wherein the vector system comprises at least two plasmids or vectors.

18. A strain according to Claim 17, wherein the selection marker of the vector system is situated on one plasmid and the remaining DNA-sequences to be incorporated in the host genome are situated on another plasmid.

19. A strain according to Claim 1, wherein the vector system comprises one plasmid or vector.

20. A method for transformation of Trichoderma, wherein a suitable Trichoderma strain is transformed with a vector system comprising:
a) a gene encoding a desired protein product,
b) functions facilitating gene expression including DNA sequences comprising promoters/enhancers operably linked to said gene to control expression of the desired product, and
c) a selection marker capable of selecting stable Trichoderma transformants, and selecting stably transformed Trichoderma through the presence of the selection marker, the selection marker not being derived from the Neurospora crassa pyr-4 gene.

21. A method for the production of a protein product in Trichoderma, wherein a strain in accordance with any one of Claims 1 to 19 is cultured in a suitable culture medium and the expressed and secreted protein product is recovered from the culture medium.

22. A method for the production of a protein product in Trichoderma which comprises cultivating in a suitable culture medium a suitable stable transformed host microorganism in accordance with any one of Claims 1 to 19 and recovering the expressed and secreted protein product from the culture medium.

23. A method in accordance with Claim 21 or 22, wherein the host microorganism is a prototrophic T. reesei strain.

24. A method in accordance with Claim 21 or 22, wherein the host microorganism is an auxotrophic T. reesei strain.

25. A method in accordance with any one of Claims 21 to 24, wherein the host strain is deficient to any gene encoding an undesired product.

26. A method according to Claim 25, wherein the host strain is deficient to the chb1 gene.

## Patentansprüche

1. Trichoderma-Stamm, der stabil transformiert ist mit einem Vektorsystem, welches umfaßt:
a) ein Gen, das ein gewünschtes Proteinprodukt kodiert,
b) Funktionen, die Genexpression erleichtern, einschließlich DNA-Sequenzen, die PromotorenNerstärker umfassen, die operabel mit besagtem Gen verbunden sind, um Expression des gewünschten Produktes zu kontrollieren, und
c) einen Selektionsmarker, der in der Lage ist, stabile Trichoderma-Transformanten zu selektieren, wobei der Selektionsmarker nicht vom Neurospora crassa pyr-4-Gen abgeleitet ist.

2. Stamm nach Anspruch 1, wobei das Vektorsystem außerdem eine Signal/Leader-Sequenz umfaßt, die stromaufwärts an das 5'-Ende des Gens für das gewünschte Proteinprodukt fusioniert ist.

3. Stamm nach Anspruch 1 oder 2, wobei der Promotor des Vektorsystems in Trichoderma funktionieren kann.

4. Stamm nach Anspruch 1, wobei der Promotor des Vektorsystems abgeleitet ist von einem Gen für ein zu Trichoderma heterologes Protein.

5. Stamm nach Anspruch 2, wobei die Signal/Leader-Sequenz des Vektorsystems abgeleitet ist von einem Gen für ein von Trichoderma sekretiertes Protein.

6. Stamm nach Anspruch 2, wobei die Signal/Leader-Sequenz des Vektorsystems abgeleitet ist von einem sekretierten Protein, das zu Trichoderma heterolog ist.

7. Stamm nach Anspruch 5 bis 6, wobei die Signal/Leader-Sequenz des Vektorsystems abgeleitet ist von der Signal/Leader-Sequenz der gewünschten Proteine.

8. Stamm nach Anspruch 5, wobei die Signalsequenz des Vektorsystems die Trichoderma reesei cbh1-Signalsequenz ist.

9. Stamm nach Anspruch 6, wobei die Signalsequenz des Vektorsystems die Aspergillus-Glucoamylase-Signalsequenz ist.

10. Stamm nach Anspruch 1, wobei das gewünschte Protein zu Trichoderma homolog ist.

11. Stamm nach Anspruch 1, wobei das gewünschte Protein zu Trichoderma heterolog ist.

12. Stamm nach Anspruch 11, wobei das gewünschte Produkt Prochymosin ist.

13. Stamm nach Anspruch 10, wobei das gewünschte Protein T. reesei-Endoglucanase (EG I) ist.

14. Stamm nach Anspruch 3, wobei der Promotor des Vektorsystems der Trichoderma reesei chb1-Promotor ist.

15. Stamm nach Anspruch 4, **dadurch gekennzeichnet, daß** der Promotor des Vektorsystems der Aspergillus-Glucoamylase-Promotor ist.

16. Stamm nach Anspruch 1, wobei der Selektionsmarker des Vektorsystems abgeleitet ist von dem Aspergillus nidulans amdS-Gen, dem Aspergillus nidulans- oder Trichoderma reesei argB-Gen, dem Trichoderma reesei pyr4-Gen oder dem Aspergillus nidulans trpC-Gen.

17. Stamm nach Anspruch 1, wobei das Vektorsystem wenigstens zwei Plasmide oder Vektoren umfaßt.

18. Stamm nach Anspruch 17, wobei der Selektionsmarker des Vektorsystems auf einem Plasmid angeordnet ist und die restlichen DNA-Sequenzen, die in das Wirtsgenom eingebaut werden sollen, auf einem anderen Plasmid angeordnet sind.

19. Stamm nach Anspruch 1, wobei das Vektorsystem ein Plasmid oder einen Vektor umfaßt.

20. Verfahren zur Transformation von Trichoderma, wobei ein geeigneter Trichoderma-Stamm mit einem Vektorsystem transformiert ist, welches umfaßt:
a) ein Gen, das ein gewünschtes Proteinprodukt kodiert,
b) Funktionen, die Genexpression erleichtern, einschließlich DNA-Sequenzen, die Promotoren/Verstärker umfassen, die operabel mit besagtem Gen verbunden sind, um Expression des gewünschten Produktes zu kontrollieren, und
c) einen Selektionsmarker, der in der Lage ist, stabile Trichoderma-Transformanten zu selektieren und stabil transformierte Trichoderma zu selektieren durch das Vorhandensein des Selektionsmarkers, wobei der Selektionsmarker nicht vom Neurospora crassa pyr-4-Gen abgeleitet ist.

21. Verfahren zur Herstellung eines Proteinproduktes in Trichoderma, wobei ein Stamm gemäß einem der Ansprüche 1 bis 19 in einem geeigneten Kulturmedium kultiviert wird und das exprimierte und sekretierte Proteinprodukt aus dem Kulturmedium gewonnen wird.

22. Verfahren zur Herstellung eines Proteinproduktes in Trichoderma, welches umfaßt, daß in einem geeigneten Kulturmedium ein geeigneter stabiler transformierter Wirtsmikroorganismus gemäß einem der Ansprüche 1 bis 19 kultiviert und das exprimierte und sekretierte Proteinprodukt aus dem Kulturmedium gewonnen wird.

23. Verfahren nach Anspruch 21 oder 22, wobei der Wirtsmikroorganismus ein prototropher T. reesei-Stamm ist.

24. Verfahren gemäß Anspruch 21 oder 22, wobei der Wirtsmikroorganismus ein auxotropher T. reesei-Stamm ist.

25. Verfahren nach einem der Ansprüche 21 bis 24, wobei dem Wirtsstamm jegliches Gen fehlt, das ein unerwünschtes Produkt kodiert.

26. Verfahren nach Anspruch 25, wobei dem Wirtsstamm das chb1-Gen fehlt.

## Revendications

1. Souche de Trichoderma transformée de façon stable avec un système vectoriel comprenant :
a) un gène codant un produit protéinique souhaité,
b) des fonctions facilitant l'expression du gène y compris les séquences ADN comprenant des promoteurs amplificateurs liés de façon utilisable sur la gène pour commander l'expression du produit souhaité, et
c) un marqueur de sélection pouvant sélectionner des transformants de Trichoderma stables, la marquer de sélection n'étant pas dérivé du gène Neurospora crassa pyr-4.

2. Souche selon le revendication 1, dans laquelle le système vectoriel comprend de plus une séquence signal/tête fusionnée en amont de l'extrémité 5' du gène pour le produit protéinique souhaité.

3. Souche selon la revendication 1 ou 2, dans laquelle le promoteur du système vectoriel peut fonctionner dans le Trichoderma.

4. Souche selon 1a revendication 1, dans laquelle le promoteur du système vectoriel est dérivé d'un gène pour protéine hétérologue ou Trichoderma.

5. Souche selon la revendication 2, dans laquelle la séquence signal/tête du système vectoriel est dérivée d'un gène pour une protéine sécrétée par le Trichoderma.

6. Souche selon la revendication 2, dans laquelle la séquence signal/tête du système vectoriel est dérivée d'une protéine sécrétée hétérologue au Trichoderma.

7. Souche selon la revendication 5 à 6, dans laquelle la séquence signal/tête du système vectoriel est dérivée de la séquence signal/tête des protéines souhaitées.

8. Souche selon la revendication 5, dans laquelle la séquence du système vectoriel est la séquence de signal Trichoderma reesei cbh1.

9. Souche selon la revendication 6, dans laquelle la séquence de signal du système vectoriel est la séquence de signal Aspergillus glucoamylase.

10. Souche selon la revendication 1, dans laquelle la protéine souhaitée est homologue au Trichoderma.

11. Souche selon la revendication 1, dans laquelle la protéine souhaitée est hétérologue au Trichoderma.

12. Souche selon la revendication 11, dans laquelle le produit souhaité est la prochymosine.

13. Souche selon la revendication 10, dons laquelle la protéine souhaitée est la T. reesei endoglucanase (EG I).

14. Souche selon la revendication 3, dans laquelle le promoteur du système vectoriel est le promoteur Trichoderma reesei chb1.

15. Souche selon la revendication 4, dans laquelle le promoteur du système vectoriel est le promoteur Aspergillus glucoamylase.

16. Souche selon la revendication 1, dans laquelle le marqueur de sélection du système vectoriel est dérivé du gène Aspergillus nidulans ambS, du gène Aspergillus nidulans ou Trichoderma reesei argB, du gène Trichoderma reesei pyr4 ou du gène Aspergillus nidulans trpC.

17. Souche selon la revendication 1, dans laquelle le système vectoriel comprend au moins deux plasmides ou vecteurs.

18. Souche selon la revendication 17, dans laquelle le marqueur de sélection du système vectoriel est situé sur un plasmide et les séquences d'ADN restant à incorporer dans le génome-hôte sont situées sur un autre plasmide.

19. Souche selon la revendication 1, dans laquelle le système vectoriel comprend un plasmide ou un vecteur.

20. Procédé pour la transformation de Trichoderma dans lequel une souche de Trichoderma appropriée est transformée avec un système vectoriel comprenant :
a) un gène codant un produit protéinique souhaité,
b) des fonctions facilitant l'expression du gène y compris les séquences d'ADN comprenant des promoteurs/amplificateurs liés de façon utilisable au gêne pour commander l'expression du produit souhaité, et
c) un marqueur de sélection pouvant sélectionner des transformants de Trichoderma stables et sélectionner le Trichoderma transformé de façon stable par la présence du marqueur de sélection, le marqueur de sélection n'étant pas dérive du gêne Neurospora crassa pyr-4.

21. Procédé pour la production d'un produit protéinique dans le Trichoderma, dans lequel uns souche selon l'une quelconque des revendications 1 à 19 est mise en culture dans un milieu de culture approprié et le produit protéinique exprimé et sécrété est récupéré du milieu de culture.

22. Procédé pour la production d'un produit protéinique dans le Trichoderma qui comprend la mise on culture dans un milieu de culture approprié d'un microorganisme-hôte approprié, transformé, stable selon l'une quelconque des revendications 1 à 19 et récupération du produit protéinique exprimé et sécréte du milieu de culture.

23. Procédé selon la revendication 21 ou 22, dans lequel le microorganisme-hôte est une souche prototrophe T. reesei.

24. Procédé selon la revendication 21 ou 22, dans lequel le microorganisme-hôte est une souche auxotrophe T. reesei.

25. Procédé selon l'une quelconque des revendications 21 à 24, dans lequel la souche-hôte est déficiente vis-à-vis de tout gène codant un produit indésiré.

26. Procédé selon la revendication 25, dans lequel la souche-hôte est déficiente vis-à-vis du gène chb1.
